(19) Europäisches Patentamt
European Patent Office
Office européen des brevets

(11) **EP 2 334 269 B1**

(12) **EUROPÄISCHE PATENTSCHRIFT**

(45) Veröffentlichungstag und Bekanntmachung des Hinweises auf die Patenterteilung:
**31.12.2014 Patentblatt 2015/01**

(21) Anmeldenummer: **09778460.7**

(22) Anmeldetag: **10.09.2009**

(51) Int Cl.:
*A61F 9/008* (2006.01)    *G06F 19/00* (2011.01)
*A61B 19/00* (2006.01)    *A61F 9/01* (2006.01)
*A61N 5/06* (2006.01)    *G06F 17/50* (2006.01)

(86) Internationale Anmeldenummer:
**PCT/EP2009/006579**

(87) Internationale Veröffentlichungsnummer:
**WO 2010/028830 (18.03.2010 Gazette 2010/11)**

(54) **STEUERPROGRAMM ZUM STEUERN ELEKTROMAGNETISCHER STRAHLUNG FÜR EINE QUERVERNETZUNG VON AUGENGEWEBE**

CONTROL PROGRAM FOR CONTROLLING ELECTROMAGNETIC RADIATION FOR CROSS-LINKING EYE TISSUE

PROGRAMME DE COMMANDE DESTINÉ À COMMANDER UN RAYONNEMENT ÉLECTROMAGNÉTIQUE LORS DE LA RÉTICULATION TRANSVERSALE DE TISSU OCULAIRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **11.09.2008 DE 102008046834**

(43) Veröffentlichungstag der Anmeldung:
**22.06.2011 Patentblatt 2011/25**

(73) Patentinhaber: **IROC Innocross AG**
**6300 Zug (CH)**

(72) Erfinder:
• **BÜELER, Michael**
**8048 Zürich (CH)**

• **MROCHEN, Michael**
**CH-8193 Eglisau (CH)**

(74) Vertreter: **von Hellfeld, Axel**
**Wuesthoff & Wuesthoff**
**Patent- und Rechtsanwälte**
**Schweigerstrasse 2**
**81541 München (DE)**

(56) Entgegenhaltungen:
WO-A1-2008/000478    WO-A2-2007/128581
US-A- 5 891 131    US-A1- 2007 161 972
US-A1- 2008 033 408    US-B1- 7 237 898

**Beschreibung**

Die Erfindung betrifft ein Steuerprogramm zum Steuern einer Quelle elektromagnetischer Strahlung mit der im Augengewebe eines Patienten eine Quervernetzung mittels eines in das Gewebe eingebrachten Photosensibilisators durchgeführt wird, sowie ein Verfahren zum Herstellen eines solchen Steuerprogramms, und ein Verfahren zum Ermitteln, ob ein Patient für eine ophthalmologische Behandlung mit Quervernetzung von Augengewebe mittels elektromagnetischer Strahlung und eines Photosensibilisators geeignet ist.

Der menschliche Augapfel wird durch die äußere Augenhaut begrenzt. Durch den Augeninnendruck wird die kollagenhaltige äußere Augenhaut gespannt und verleiht dem gesunden Augapfel eine annähernd sphärische Form. Im hinteren Augapfelbereich besteht die äußere Augenhaut aus der weißen Lederhaut (Sklera). Im vorderen Bereich befindet sich die für sichtbares Licht durchlässige Hornhaut (Kornea).

Verformungen der äußeren Augenhaut können Ursache einer Fehlsichtigkeit sein. Beispielsweise kann eine Form der Kurzsichtigkeit, die Achsenmyopie, Folge eines skleralen Längenwachstums des Augapfels sein. Eine als Ellipsoid geformte Oberfläche der Kornea kann zu einer Form des Astigmatismus führen, welche auch als Hornhautverkrümmung bezeichnet wird. Eine weitere Erkrankung der Hornhaut wird als Keratokonus bezeichnet. Beim Keratokonus kommt es in Folge einer krankhaften Erweichung der Hornhaut zu einer fortschreitenden Ausdünnung und kegelförmigen Verformung der Hornhaut des Auges. Mit der zunehmenden Auswölbung wird die Hornhaut unterhalb des Zentrums dünner. Sie kann durchbrechen und vernarben. Dies setzt die Sehschärfe dauerhaft herunter. Die Ursachen des Keratokonus sind heutzutage noch weitgehend unbekannt. Er tritt familiär gehäuft auf, was unter anderem für eine genetische Disposition spricht. Einen weiteren Risikofaktor für die Entstehung eines Keratokonus stellen Atopien, wie allergische Erkrankungen, dar.

Die konventionelle Therapie eines fortgeschrittenen Keratokonus sieht vor, die erkrankte Kornea zu entfernen und durch ein allogenes Transplantat zu ersetzen. Eine solche Operation ist jedoch eine Organverpflanzung mit den damit verbundenen Risiken und Komplikationen. Ein angemessenes Sehvermögen wird häufig erst ca. zwei Jahre nach der Operation erreicht. Zudem betrifft die Hornhautverpflanzung beim Keratokonus meist junge Menschen, weshalb das Transplantat über Jahrzehnte hinweg einwandfrei funktionieren muss.

Eine demgegenüber verbesserte Therapie des Keratokonus stabilisiert die Kornea durch Quervernetzung. Die Behandlung erlaubt eine photochemische, nichtgewebeabtragende Stabilisierung oder Veränderung der biomechanischen und biochemischen Eigenschaften der Kornea. Das Behandlungsprinzip ist auch auf andere betroffene Regionen des Auges anwendbar. Eine Photosensibilisatorlösung wird in das zu verändernde Augengewebe eingebracht und einer Primärstrahlung ausgesetzt. Als Primärstrahlung wird elektromagnetische Strahlung im Wellenlängebereich von etwa 300 nm bis 800 nm (UV-A-Strahlung oder sichtbares Licht) eingesetzt.

Entsprechende Vorrichtungen zur Behandlung der äußeren Augenhaut sind aus den Druckschriften WO 2007/128581 A2 und WO 2008/000478 A1 bekannt.

Eine Vorrichtung gemäß WO 2007/128581 A2 dient der Verfestigung der sich im hinteren Augenabschnitt befindenden Sklera. Die Primärstrahlung kann dabei durch das Innere des Auges oder durch von außen aufliegenden Kissen auf die Sklera einwirken. Durch einen Photomediator oder Photosensibilisator wird eine Quervernetzung in der Sklera bewirkt. Dadurch wird einem Sklerawachstum entgegengewirkt und ein Fortschreiten der Achsenmyopie unterbunden.

Die Druckschrift WO 2008/000478 A1 beschreibt ein Bestrahlungssystem zur biomechanischen Stabilisierung der Kornea. Auch hier kann in Verbindung mit einem Photosensibilisator eine Quervernetzung an der Kornea bewirkt werden. Das Bestrahlungssystem bietet die Möglichkeit spezifische Erkrankungen, wie den Keratokonus zu therapieren.

Die Druckschrift US 2008/033408 A1 beschreibt ein Computerprogramm zum Bestimmen eines Veränderungsprofils für die refraktive Augenchirurgie mittels eines Lasersystems. Das Computerprogramm umfasst eine Benutzerschnittstelle zur Eingabe von Daten durch einen Benutzer und einer Datenaufnahmeschnittstelle für die Aufnahme von Messdaten. Das Computerprogramm generiert auf Basis der eingegebenen Daten und Messdaten ein Veränderungsprofil, das zum Steuern des Lasersystems herangezogen wird.

Die Druckschrift WO 02/07660 A2 beschreibt ein Verfahren zum laserchirurgischen Eingriff gemäß photorefraktiver Keratektomie (PRK) oder einer lasergestützten in situ-Keratomileusis (LASIK) unter Berücksichtigung einer biomechanischen Reaktion auf die durch Laserablation bewirkte Strukturänderung.

Die Änderung der Gestalt und/oder von mechanischen Eigenschaften von Augengewebe, insbesondere der Hornhaut und allgemein der Sklera mittels eines eingebrachten Photosensibilisators und elektromagnetischer Strahlung ist als solche im Stand der Technik, insbesondere wie oben genannt, gut bekannt. Hinsichtlich der chemischen Zusammensetzung des Photosensibilisators wird auf den Stand der Technik verwiesen, auch hinsichtlich der eingesetzten Art der elektromagnetischen Strahlung, insbesondere der geeigneten Wellenlängen in Verbindung mit bestimmten Photosensibilisatoren.

Einem routinemäßigen Einsatz der Quervernetzungstherapie am Augengewebe stehen jedoch komplexe Abhängigkeiten entgegen. Die Beziehungen zwischen den eingesetzten Dosen und deren Wirkung im Augengewebe sind sehr vielfältig. Als Dosis in diesem Sinne kommen insbesondere in Betracht die elektromagnetische Strahlung hinsichtlich ihrer Intensität sowie ihrer Verteilung in Raum und Zeit; der eingesetzte Photosensibilisator hinsichtlich seiner chemischen Struktur, Konzentration, und Einwirkung in Raum und Zeit. Die Wirkungen von unterschiedlichen Dosen dieser Parameter auf und in dem Augengewebe eines Patienten sind sehr stark abhängig von Eigenschaften (Messdaten) bezüglich des Patienten. Dabei ist insbesondere zu berücksichtigen, dass die Wirkung der mit der Strahlung und dem Photosensibilisator durchgeführten Quervernetzung auch unerwünscht sein kann bis hin zu einer Schädigung des Augengewebes oder der Funktion des Auges.

Der Erfindung liegt die Aufgabe zugrunde, Verfahren und Steuerprogramme bereitzustellen, mit denen bereits im Vorfeld eines eventuellen ophthalmologischen Eingriffs eine Beurteilung der Wirkung einer Quervernetzung möglichst zuverlässig abschätzbar ist.

Hierzu stellt die Erfindung bereit ein Verfahren zum Erzeugen eines Steuerprogramms zum Steuern einer Quelle elektromagnetischer Strahlung, mit der im Augengewebe eines Patienten eine Quervernetzung mittels eines in das Gewebe eingebrachten Photosensibilisator durchgeführt wird, um eine Änderung des Augengewebes bezüglich seiner Gestalt und/oder zumindest einer seiner mechanischen oder optischen Eigenschaften zu bewirken, mit folgenden Schritten:

a) es werden Messdaten bezüglich des Patienten und des Auges gewonnen,

b) in einem Rechner wird mit den Messdaten und Behandlungsparametern, die ein vorgegebenes Hilfs-Steuerprogramm zum Steuern der Quelle elektromagnetischer Strahlung beinhalten, ein Ergebnis bezüglich der mit diesen Behandlungsparametern erreichten Änderung des Augengewebes simuliert durch

- Simulieren einer Diffusion des Photosensibilisators zur Bestimmung einer Verteilung des Photosensibilisators und

- Bestimmen einer tiefenabhängigen Absorption der Strahlung aufgrund der simulierten Verteilung des Photosensibilisators,

c) die so simulierte Änderung wird mit einer zu erzielenden Änderung des Augengewebes verglichen,

d) dann, wenn Schritt c) eine hinreichende Übereinstimmung zwischen der simulierten Änderung und der zu erzielenden Änderung des Augengewebes ergibt, wird das in Schritt b) angesetzte Hilfs-Steuerprogramm als das zu erzeugende Steuerprogramm gewählt, und

e) dann, wenn in Schritt c) der Vergleich keine hinreichende Übereinstimmung zwischen der simulierten Änderung und der zu erzielenden Änderung des Augengewebes ergibt, werden zumindest einer der Behandlungsparameter in Schritt b) abgeändert und damit dann die Schritte b), c) und d) erneut durchgeführt.

Gemäß einer bevorzugten Ausgestaltung ist vorgesehen, dass in Schritt a) als Messdaten eine oder mehrere der folgenden Daten herangezogen werden: die Hornhaut-Topographie des Auges des Patienten; die Form der Hornhautrückfläche; andere Formeigenschaften der Vorder- und/oder Rückfläche der Hornhaut, wie Vertiefungen oder Erhöhungen, biomechanische Eigenschaften der Hornhaut, wie deren Stabilität; die Hornhautdicke; die Augenlänge; die Tiefe und/oder das Volumen der Vorderkammer; Ergebnisse von Wellenfrontmessungen; das Alter; Geschlecht; hormoneller Status des Patienten; der Zustand einer gegebenen Ektasie; Lebensgewohnheiten (z.B. Rauchen) des Patienten.

Eine andere bevorzugte Ausgestaltung des Verfahrens sieht vor, dass in Schritt b) das Hilfs-Steuerprogramm eine oder mehrere der folgenden Größen berücksichtigt: Intensität der Bestrahlung mit der elektromagnetischen Strahlung; Konzentration des Photosensibilisators im Gewebe; die osmotische Charakteristik des Photosensibilisators; die Bestrahlungszeit; die Zeitspanne der Applikation des Photosensibilisators vor der Bestrahlung; die Zeitspannen der Applikation des Photosensibilisators während der Bestrahlung; die Art der elektromagnetischen Strahlung (z.B. kontinuierlich oder gepulst) einschließlich der Strahlungsparameter, wie die Wellenlänge; die Tastrate bei gepulster Strahlung; die räumliche und zeitliche Steuerung der Strahlung.

Weiterhin stellt die Erfindung Computerprogrammprodukt bereit zum Bestimmen von Parametern eines Steuerprogramms, wobei das Steuerprogramm ausgebildet ist zum Steuern von elektromagnetischer Strahlung zur Durchführung einer Quervernetzung in Augengewebe, wobei das Computerprogrammprodukt Programmcode umfasst, welcher geeignet ist, die folgenden Schritte durchzuführen, wenn das Computerprogrammprodukt auf einer Computeranlage ausgeführt wird:

a') es werden Messdaten bezüglich des Patienten und des Auges gewonnen,

b') in einem Rechner wird mit den Messdaten und Behandlungsparametern, die ein vorgegebenes Hilfs-Steuerprogramm zum Steuern der Quelle elektromagnetischer Strahlung beinhalten, ein Ergebnis bezüglich der mit diesen Behandlungsparametern erreichten Änderung des Augengewebes simuliert durch

- Simulieren einer Diffusion des Photosensibilisators zur Bestimmung einer Verteilung des Photosensibilisators und

- Bestimmen einer tiefenabhängigen Absorption der Strahlung aufgrund der simulierten Verteilung des Photosensibilisators,

c') die so simulierte Änderung wird mit einer zu erzielenden Änderung des Augengewebes verglichen,

d') dann, wenn Schritt c') eine hinreichende Übereinstimmung zwischen der simulierten Änderung und der zu erzielenden Änderung des Augengewebes ergibt, wird das in Schritt b') angesetzte Hilfs-Steuerprogramm als das zu erzeugende Steuerprogramm gewählt, und

e') dann, wenn in Schritt c') der Vergleich keine hinreichende Übereinstimmung zwischen der simulierten Änderung und der zu erzielenden Änderung des Augengewebes ergibt, werden zumindest einer der Behandlungsparameter in Schritt b') abgeändert und damit dann die Schritte b'), c') und d') erneut durchgeführt.

Ferner kann gemäß einem besonders einfachen Verfahren ermittelt werden, ob ein Patient überhaupt für einen ophthalmologischen Eingriff mittels Quervernetzung geeignet ist. Hierzu kann ein Verfahren angewendet werden zum Ermitteln, ob ein Patient für eine ophthalmologische Behandlung unter Quervernetzung von Augengewebe mittels elektromagnetischer Strahlung und mittels eines Photosensibilisators geeignet ist, mit folgenden Schritten:

a") Es können Messdaten bezüglich des Patienten und des Auges gewonnen werden,

b") in einem Rechner kann mit den Messdaten und Behandlungsparametern, die ein vorgegebenes Hilfs-Steuerprogramm zum Steuern der Quelle elektromagnetischer Strahlung beinhalten, ein Ergebnis bezüglich der mit diesen Behandlungsparametern erreichten Änderung des Augengewebes simuliert werden,

c") es kann zumindest eine kritische Änderung des Augengewebes vorgegeben werden,

d") es kann die simulierte Änderung des Augengewebes mit der kritischen Änderung des Augengewebes verglichen werden und

e") dann, wenn die simulierte Änderung hinreichend mit der kritischen Änderung übereinstimmt, kann festgestellt werden, dass der Patient nicht für eine

d") es wird die simulierte Änderung des Augengewebes mit der kritischen Änderung des Augengewebes verglichen und

e") dann, wenn die simulierte Änderung hinreichend mit der kritischen Änderung übereinstimmt, festgestellt, dass der Patient nicht für eine ophthalmologische Behandlung unter Verwendung des Hilfs-Steuerprogramms geeignet ist.

In einer Weiterbildung sieht das Hilfs-Steuerprogramm vor, zeitgleich mit oder nach dem Steuern der Quelle elektromagnetischer Strahlung zur Quervernetzung ein (zweites) Steuern einer Quelle elektromagnetischer Strahlung zur Änderung des Augengewebes bezüglich seiner Gestalt und/oder zumindest einer seiner mechanischen Eigenschaften durch photothermische Wirkung, Photoablation und/oder Photodisruption vor. Insbesondere kann die genannte Quelle elektromagnetischer Strahlung zur photothermischen Wirkung, Photoablation und/oder Photodisruption ein Femtosekundenlaser oder ein Exzimerlaser sein.

Nachfolgend werden Ausführungsbeispiele der Erfindung näher beschrieben.

Es zeigt:

Figur 1     schematisch ein Blockdiagramm für die Ausführung der Erfindung in ihrem vollen Umfang;

Figur 2      schematisch ein Blockdiagramm der Ausführung der Erfindung in eingeschränktem Umfang;

Figur 3      ein Blockdiagramm zur Veranschaulichung des erfindungsgemäßen Verfahrens zum Erzeugen eines Steuerprogramms für die Steuerung von elektromagnetischer Strahlung bei einer Quervernetzung;

Figur 4      schematisch einen empirisch beobachteten Zusammenhang hinsichtlich Lichtabschwächung durch Photosensibilisator und Hornhaut als Funktion einer Hornhauttiefe;

Figur 5      schematisch eine gemessene Zunahme einer biomechanischen Festigkeit der Hornhaut als Funktion einer berechneten Zunahme der Polymerzahl in der Hornhaut; und

Figur 6      schematisch eine beobachtete Brechkraftänderung der Hornhaut ein Jahr nach Cross-Linking als Funktion eines Vorhersagekriteriums aus gemessenen prä-operativen Größen.

Unter einer Quervernetzung (englisch: Cross-Linking) versteht man in der Ophthalmologie eine photochemische Beeinflussung von Augengewebe. Die durch diese Beeinflussung erzielte Veränderung kann in den biomechanischen und biochemischen Eigenschaften des Gewebes liegen, insbesondere aber auch in einer Veränderung der Gestalt des Gewebes, insbesondere seiner Form und/oder Krümmung. Auch eine Stabilisierung des Gewebes wird durch eine Quervernetzung erreicht, d.h. eine Änderung seiner mechanischen Elastizität. Hierzu wird in an sich bekannter Weise ein Photosensibilisator, d.h. eine geeignete chemische Lösung auf und in das Augengewebe gebracht, welche dann zusammen mit der elektromagnetischen Strahlung die photochemischen Prozesse im Gewebe bewirkt, die unter dem Stichwort "Quervernetzung" angesprochen werden. Dabei ist die Wirkung der elektromagnetischen Strahlung, auch "Primärstrahlung" genannt, am und im Gewebe weder thermisch noch photoablativ, noch photodisruptiv.

Die nachfolgend näher beschriebene Erfindung stellt ein Verfahren zur Verfügung, mit dem die komplexen Beziehungen zwischen den diversen Eigenschaften der eingesetzten elektromagnetischen Strahlung (z.B. Wellenlänge, Intensität, Verteilung in Raum und Zeit) sowie den diversen Eigenschaften des eingesetzten Photosensibilisators (z.B. chemische Natur; Konzentration; Verdünnung; osmotische Eigenschaften; Zeitfolge des Aufbringens etc.) und der damit erzielten Wirkung im Augengewebe, insbesondere der Hornhaut, für jeden individuellen Patienten zumindest annähernd bestimmt werden können. Der vollumfängliche Einsatz der Erfindung ermöglicht die Gewinnung eines Steuerprogramms, mit dem elektromagnetische Strahlung für die Quervernetzung in das Augengewebe eingebracht werden kann. Ein Steuerprogramm in diesem Sinne beinhaltet die im Stand der Technik als solches bekannte Steuerung von optischen Mitteln, mit denen Laserstrahlung z.B. über Spiegel und andere abbildende optische Elemente, wie Linsen etc., auf das zu behandelnde Auge gerichtet werden kann, z.B. in Form eines sogenannten "Spots", also eines im Verhältnis zu den Gesamtabmessungen des Auges relativ kleinen Flecks (mit einem Durchmesser im mm-Bereich), der gemäß einem vorgegebenen Programm in Raum und Zeit in Bezug auf das Auge geführt wird. Es sind auch im Stand der Technik andere Mittel bekannt, um elektromagnetisch Strahlung gemäß einem gewünschten Programm in Augengewebe einzubringen, wie z.B. rotierende Masken, bewegte Blenden etc.

Insbesondere ist als Vorrichtung zur Ausführung der vorliegenden Erfindung und zur Steuerung von elektromagnetischer Strahlung auf ein Auge das Bestrahlungssystem gemäß der internationalen Patentanmeldung PCT/EP2007/005740 (oben genannte Veröffentlichung WO 2008/000478 A1) einsetzbar. Dieses bekannte Bestrahlungssystem für ophthalmologische Anwendungen wird für die Ausführung der vorliegenden Erfindung als ausdrücklich bekannt vorausgesetzt.

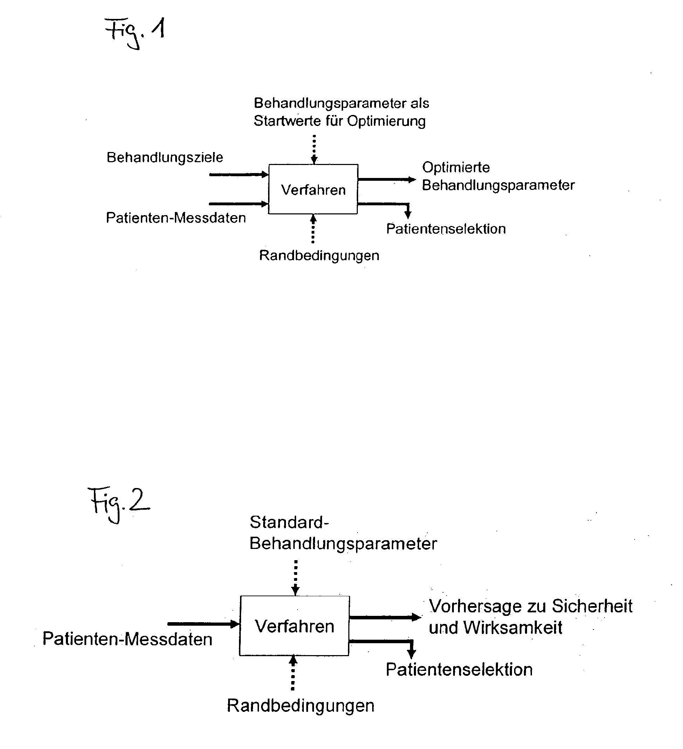

Figur 1 illustriert schematisch die Eingangsgrößen für das erfindungsgemäße Verfahren zum Erzeugen eines Steuerprogramms und die nach Durchführung des Verfahrens erhaltenen Ergebnisse. Das Verfahren ist bei voller Ausführung iterativ, d.h. ausgehend von einem relativ allgemein gültigen Hilfs-Steuerprogramm wird dieses anfängliche Hilfs-Steuerprogramm sukzessive, Schleife für Schleife (also iterativ), solange verbessert, bis die Eigenschaften des so erzeugten Steuerprogramms den gewünschten Erwartungen unter Berücksichtigung der Eigenschaften des Patienten und seines Auges entsprechen.

Eingang in das Verfahren zum iterativen Erzeugen des genannten Steuerprogramms finden zunächst gemäß Figur 1 "Behandlungsparameter" als Startwerte für die Optimierung. Diese Behandlungsparameter beinhalten unter anderem das vorstehend genannte anfängliche Hilfs-Steuerprogramm, mit dem der Iterationsprozess begonnen werden kann.

Weitere Eingabedaten in den das Verfahren ausführenden und entsprechend programmierten Rechner sind die "Behandlungsziele". Behandlungsziele sind z.B. die gewünschten therapeutischen Änderungen in der Gestalt und/oder den mechanischen Eigenschaften des zu behandelnden Augengewebes, insbesondere der Kornea.

Zu den "Behandlungsparametern" im genannten Sinne gehören insbesondere folgende Größen, die das Hilfs-Steuerprogramm als zu berücksichtigende Daten verwendet: Intensität der Bestrahlung mit der elektromagnetischen Strahlung; Konzentration des Photosensibilisators im Gewebe; die osmotische Charakteristik des Photosensibilisators;

die Bestrahlungszeit; die Zeitspanne der Applikation des Photosensibilisators vor der Bestrahlung; die Zeitspannen der Applikation des Photosensibilisators während der Bestrahlung; die Art der elektromagnetischen Strahlung (z.B. kontinuierlich oder gepulst) einschließlich der Strahlungsparameter, wie Wellenlänge; die Tastrate bei gepulster Strahlung; die räumliche und zeitliche Steuerung der Strahlung.

Weitere Eingabedaten in den programmierten, das Verfahren ausführenden Rechner sind "Patienten-Messdaten". Dies sind insbesondere folgende Daten: die Hornhaut-Topographie des Auges des Patienten; die Form der Hornhautrückfläche; andere Formeigenschaften der Vorder- und/oder Rückfläche der Hornhaut, wie Vertiefungen oder Erhöhungen; biomechanische Eigenschaften der Hornhaut, wie deren Stabilität; die Hornhautdicke; die Augenlänge; die Tiefe und/oder das Volumen der Vorderkammer; Ergebnisse von Wellenfrontmessungen; das Alter, Geschlecht, hormoneller Status des Patienten; der Zustand einer gegebenen Ektasie; Lebensgewohnheiten (z.B. Rauchen) des Patienten. Die vorstehend genannten Daten können jeweils einzeln oder in beliebiger Kombination als "Patienten-Messdaten" eingegeben werden.

Weiterhin nimmt das Hilfs-Steuerprogramm anfänglich sogenannte "Randbedingungen" als Eingabedaten auf. Dies sind insbesondere sicherheitsrelevante Daten, d.h. Vorgaben, die das Programm bei seiner weiter unten näher beschriebenen Ausführung berücksichtigt, um unerwünschte Effekte im behandelten Gewebe zu vermeiden. Dies sind insbesondere Zellschädigungen am Endothel der Hornhaut und die Vermeidung eines sogenannten "Over-Cross-Linking"-Effektes im Stroma. Wenn das Programm aufgrund der Eingabedaten einschließlich der Patientendaten bei der Simulation des damit bewirkten Behandlungsergebnisses zu dem Ergebnis kommt, dass z.B. eine Zellschädigung am Endothel oder ein Over-Cross-Linking zu befürchten ist, dann wird dies in den Ausgabedaten dem Benutzer deutlich mitgeteilt, insbesondere auch in Form einer besonderen Warnung.

Weiterhin werden in das Programm die "Behandlungsziele" eingegeben, d.h. die mit der Quervernetzung zu erzielenden Änderungen des Augengewebes, um einen therapeutischen Effekt zu erzielen. Die Behandlungsziele bestehen also in einer gewünschten Änderung des Augengewebes hinsichtlich seiner Gestalt (Form/Krümmung) und/oder seiner biomechanischen Eigenschaften, wie Festigkeit und Stabilität.

Eine besonders herauszustellende Anwendung stellen Behandlungsziele dar, die auf eine selektive biomechanische Verformung der Hornhaut zur Korrektur von Fehlsichtigkeiten gerichtet sind. In diesem Fall werden Formänderungen der Hornhaut bzw. Gewebestruktur durch die Behandlungsziele vorgegeben und durch korrespondierende Zielgrößen des Hilfs-Steuerprogramms in der Simulation berücksichtigt. Diese Veränderung in der Gestalt oder den mechanischen Eigenschaften des Augengewebes zur Korrektur von Fehlsichtigkeiten geht vorteilhafterweise über die rein photochemische Quervernetzung hinaus und schließt auch eine photothermische Wirkung, eine Photoablation und/oder Photodisruption, induziert durch elektromagnetische Strahlung (korrektive Bestrahlung), ein. Vorteilhafterweise erfordert diese Erweiterung keine aufwändigen Umbauten am Bestrahlungssystem, sondern ist allein durch eine entsprechende Erweiterung des Steuerprogramms zu erreichen. Die Hilfs-Steuerprogramme, und damit letztlich auch die Steuerprogramme, können die korrektive Bestrahlung vorhergehend, gleichzeitig mit oder nachfolgend der photochemischen Behandlung des Gewebes durch Quervernetzung zeitlich einordnen. In einer erweiterten Ausführungsform enthält das (Hilfs-)Steuerprogramm Parameter und/oder Steuersignale zur Ansteuerung eines Femtosekundenlasers oder eines Exzimerlasers für die korrektive Behandlung.

In Abhängigkeit von den vorstehend genannten Eingabedaten in das anfängliche Hilfs-Steuerprogramm beginnt dieses sodann die Wirkungen in Abhängigkeit von den eingegebenen Daten zu berechnen. Berechnungsziele sind dabei insbesondere: Keratozytenschädigungstiefe, das Auftreten des genannten Over-Cross-Linking und die dazugehörige Gewebetiefe, die mechanische Festigkeitszunahme in Abhängigkeit von der Gewebetiefe, eine Gestaltänderung des bestrahlten Gewebes hinsichtlich Form und Krümmung direkt nach der Behandlung, den im Anschluss an die Behandlung erfolgenden zeitlichen Verlauf der vorstehend genannten Änderungen hinsichtlich Festigkeit und Form des Gewebes (diese Eigenschaften können sich direkt nach der Behandlung anders darstellen als Stunden oder Tage nach der Behandlung).

Eine Möglichkeit, das Steuerprogramm zu programmieren, besteht darin, empirisch den Zusammenhang zwischen den genannten Eingabedaten (also grob gesagt: Patientendaten; Strahlungsdaten; Photosensibilisatordaten (näheres siehe oben)) und der damit erzielten Änderung (Wirkung) im Augengewebe zu ermitteln und in der Art einer "Look-up-Tabelle" im Programm abzulegen. Dies ist möglich unter Einsatz sowohl einer Vielzahl von geeigneten Versuchstieren als auch unter Einsatz von Daten die mit einer Vielzahl von Patienten gewonnen werden.

Eine weitere, gegebenenfalls ergänzende Möglichkeit, im Steuerprogramm die jeweiligen Wirkungen (Änderungen des Augengewebes) in Abhängigkeit von den Eingabedaten zu simulieren sieht die Heranziehung von theoretischen Konzepten vor. Dies sind insbesondere physikalische Modelle für den Photopolymerisationsprozess (die Quervernetzung) in Form mathematischer Beschreibungen der einzelnen Teilprozesse. Solche physikalischen Modelle ermöglichen beispielsweise die Simulation der Diffusion der Photosensibilisatorlösung in das Augengewebe mittels der Diffusionsgleichungen. Auch kann die Wirkung der elektromagnetischen Strahlung modellhaft berechnet werden indem die Absorption der Photonen im Gewebe und in der Photosensibilisatorlösung berechnet wird, und zwar mittels der als solches bekannten Absorptionsgleichungen in Abhängigkeit von den Wirkungsquerschnitten. Weiterhin können solche theoretischen Verfahren, die als solche bekannt sind, eingesetzt werden, um die Änderung der Gestalt des Augengewebes zu simulieren in Abhängigkeit von der erzielten Änderung der mechanischen Festigkeit, z.B. durch Einsatz der in der Mechanik gut bekannten Methode finiter Elemente.

Mit diesen teilweise oder gänzlich empirischen Hilfsmitteln berechnet das Programm die Wirkung der elektromagnetischen Strahlung unter Berücksichtigung der Eingabedaten und es kann sodann entweder im Programm oder durch den Benutzer eine Abschätzung erfolgen, ob die simulierte Änderung der Eigenschaften des Augengewebes mit den gewünschten therapeutischen Änderungen des Augengewebes hinreichend übereinstimmt. Für die "hinreichende Übereinstimmung" können dann jeweils Toleranzwerte bezüglich der einzelnen Parameter vorgegeben werden und dann, wenn die Abweichung innerhalb der Toleranzwerte liegt, kann auf "hinreichende Übereinstimmung" erkannt werden.

Wird eine hinreichende Übereinstimmung erkannt, dann kann das zuletzt verwendete Steuerprogramm herangezogen werden als das gesuchte zu erzeugende Steuerprogramm zum Steuern der elektromagnetischen Strahlung und es kann die Behandlung des Patienten damit durchgeführt werden.

Liegt der Unterschied zwischen dem simulierten Ergebnis der Änderung des Augengewebes und der gewünschten therapeutischen Änderung des Augengewebes außerhalb der genannten Toleranzwerte, dann werden die Behandlungsparameter, z.B. das für die nächste Schleife eingesetzte Hilfs-Steuerprogramm, geändert. Zum Beispiel können hinsichtlich des Photosensibilisators dessen Konzentration, zeitlicher Auftrag, chemische Natur, etc. geändert werden oder es können Eigenschaften der im Simulationsprozess eingesetzten elektromagnetischen Strahlungen abgeändert werden, wie z.B. Bestrahlungsintensität, Bestrahlungszeit, Art der Strahlung (kontinuierlich oder gepulst) einschließlich der entsprechenden Tastrate etc., die Wellenlänge der Strahlung, die Verteilung der Strahlung in Raum und Zeit. Die Richtung der Änderung dieser Eingangs-Behandlungsparameter kann im Programm auch aufgrund von empirischen Erfahrungen vorgegeben werden, z.B. bei dem Ergebnis eines Over-Cross-Linking in der vorangegangenen Schleife kann die Bestrahlungsintensität und/oder die Photosensibilisatorkonzentration verringert werden.

Im Ergebnis liefert das iterative Verfahren, gegebenenfalls nach Durchlaufen mehrerer Simulationsschleifen, optimierte Behandlungsparameter einschließlich des Steuerprogramms zum Steuern der elektromagnetischen Strahlung, d.h. ein Programm, das alle wesentlichen Parameter der elektromagnetischen Strahlung, wie Intensität, Verteilung in Raum und Zeit, Wellenlänge, Tastrate etc. beinhaltet. Auch liefert das Programm optimale Werte bezüglich der Parameter des Photosensibilisators, wie chemische Struktur, Konzentration, zeitlicher Auftrag, räumlicher Auftrag etc.

Als Ergebnis des beschriebenen Iterationsverfahrens im Rechner kann auch herauskommen, dass der Patient, dessen Daten zu den Eingabedaten zählen, nicht für die ophthalmologische Behandlung mit Quervernetzung geeignet ist. Das ist in Figur 1 mit dem Stichwort "Patientenselektion" bezeichnet.

Figur 2 zeigt eine vereinfachte Variante der Erfindung, die es ermöglicht, für einen gegebenen Patienten zu beurteilen, ob er für eine Behandlung der hier in Rede stehenden Art mit Quervernetzung überhaupt geeignet ist, und zwar unter Verwendung vorgegebener Standard-Behandlungsparameter. Das Verfahren ist dann nicht iterativ, sondern besteht aus einer einzigen Rechenschleife unter Verwendung der Standard-Behandlungsparameter, der individuellen Messdaten des Patienten und Randbedingungen im obigen Sinn. Damit wird dann, wie oben, die Änderung des Augengewebes durch Simulation berechnet und aufgrund des simulierten Ergebnisses wird eine Aussage getroffen, ob der Patient für eine Behandlung mit Quervernetzung geeignet ist. Hierfür werden insbesondere die oben genannten Randbedingungen herangezogen und es wird festgestellt, ob das Simulationsergebnis hinsichtlich der Randbedingungen kritische Werte erreicht.

So kann z.B. für einen bestimmten Patienten bei Anwendung der Standard-Behandlungsparameter eine Keratozytenschädigungstiefe durch Simulation sich ergeben, welche eine Behandlung des Patienten durch Quervernetzung mit den Standard-Behandlungsparametern verbietet. Auch kann bei Einsatz der Standard-Behandlungsparameter sich eine Krümmungsänderung der Hornhaut bei der Simulation ergeben, welche weit entfernt ist vom gewünschten Behandlungsziel. Dies ermöglicht einem Anwender mit relativ geringer Ausstattung an Hardware und Software ohne Möglichkeit der Änderung der Eingabe-Behandlungsparameter eine Auswahl von für die Behandlung geeigneten Patienten. Eine Beschränkung auf die Standard-Behandlungsparameter kann auch durch einen Erfahrungshorizont des Operateurs oder aus zulassungsrechtlichen Gründen geboten sein.

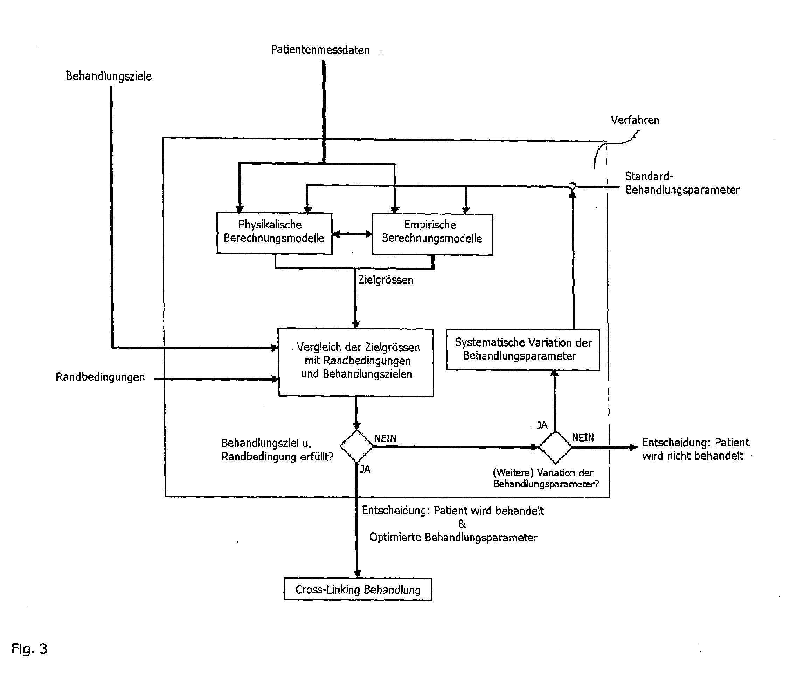

Figur 3 fasst noch einmal schematisch in einem Blockdiagramm die wesentlichen Merkmale der Erzeugung des Steuerprogramms zum Steuern der Strahlung für das Quervernetzen zusammen. Das Diagramm illustriert in Kurzfassung die oben beschriebenen Verfahren zur Steuerprogrammerzeugung. Explizite Beispiele für die im Diagramm genannten Größen sind nachfolgender Tabelle zu entnehmen.

| Größe | Beispiele |
|---|---|
| Behandlungsziele | max. Festigkeitszunahme, min. Behandlungszeit, bestimmte Formänderung etc. |

(fortgesetzt)

| Größe | Beispiele |
|---|---|
| Patientenmessdaten | Topographie von Hornhautvorder- und -rückfläche, biomech. Stabilität der Hornhaut, Hornhautdicke, Augenlänge, Vorderkammertiefe- und -volumen, Augenlänge, Wellenfront, Alter, Geschlecht etc. |
| Standard-Behandlungsparameter | Startwerte für Optimierung |
| Zielgrössen | Keratozytenschädigungstiefe, mechanische Festigkeitszunahme, Form-/Krümmungsänderung des Gewebes etc. |
| Randbedingungen | max. Keratozytenschädigungstiefe 100 Mikron von Endothel entfernt, kein Over-Cross-Linking-Effekt etc. |
| Optimierte Behandlungsparameter | Bestrahlungsintensität, Photosensitizerkonzentration, osmotische Charakteristik der Phtosensitizerkonzentration, Bestrahlungszeit, Photosensitizer-Applikationszeit vor der Bestrahlung, Photosensitizer-Applikationsintervalle während der Bestrahlung, die Art der Strahlung (kontinuierlich oder gepulst), Wellenlänge, Intensitätsverteilung bzw. Bestrahlungspattern der Strahlung etc. |

Nachstehend werden die dargestellten Verfahren sowie die physikalischen und empirischen Berechnungsmodelle näher erläutert.

Das Verfahren geht von den patientenspezifischen Messdaten aus, welche Eingang in die physikalischen und empirischen Berechnungsmodelle finden. Der zu simulierende Datensatz wird anfänglich durch die Standard-Behandlungsparameter vervollständigt.

Als Behandlungsziele werden dem Verfahren eines der folgenden Ziele oder eine Kombination folgender Ziele vorgegeben: Maximierung einer mechanischen Festigkeitszunahme des Augengewebes, Minimierung einer Zeit einer Behandlung des Augengewebes, Verformung des Augengewebes gemäß einer vorgegebenen Struktur und definierte Änderung eines Brechungsindex im Augengewebe. Diesen Behandlungszielen werden die durch die Simulation berechnete mechanische Festigkeitszunahme des Augengewebes, die Verformung des Augengewebes direkt nach der Behandlung, die Verformung des Augengewebes in Abhängigkeit von der Zeit der Behandlung bzw. der Brechungsindex im Augengewebe gegenübergestellt. Hierbei werden insbesondere die mechanische Festigkeitszunahme und der Brechungsindex in Abhängigkeit einer Tiefe im Augengewebe berechnet. Ferner werden dem Verfahren sicherheitsbedingte Randbedingungen vorgeschrieben. Diese schließen beispielsweise eine Schädigung von Keratozyten an einem Endothel des Augengewebes und einen Effekt einer zu weit gehenden Quervernetzung ("Over-Cross-Linking") in Stroma des Augengewebes aus. Diesen geforderten Randbedingungen werden die durch die Simulation berechnete Tiefe der Schädigung der Keratozyten und der Tiefe des Effekts der Überquervernetzung gegenübergestellt.

Der Berechnung werden folgende physikalische Modelle zugrunde gelegt. Zunächst wird die Diffusion des Photosensibilisators mit Konzentration c zeitabhängig berechnet. Dies geschieht gemäß der Diffusionsgleichung

$$\frac{\partial c}{\partial t} = D\left(\frac{\partial^2 c}{\partial x^2} + \frac{\partial^2 c}{\partial y^2} + \frac{\partial^2 c}{\partial z^2}\right),$$

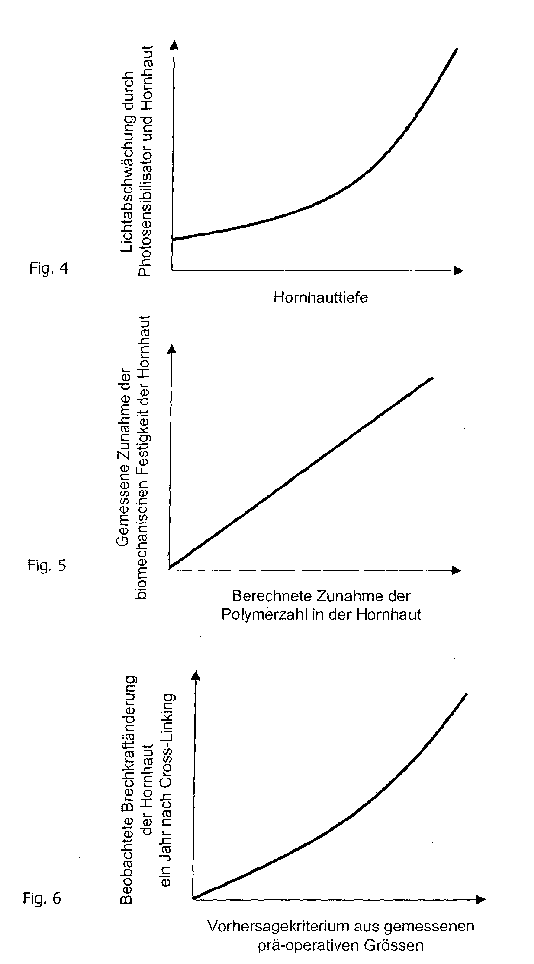

welche unter Berücksichtigung der Oberflächengeometrie der Hornhaut numerisch gelöst wird. Damit ist die Verteilung des Photosensibilisators bekannt. Nun wird die Strahlungsabsorption durch die Hornhaut und die bekannte Verteilung des Photosensibilisator gemäß dem wellenlängenabhängigen Lambert-Beer'schen Gesetz, vorzugsweise unter Berücksichtigung des empirisch gefundenen, in Fig. 4 schematisch gezeigten Zusammenhangs, bestimmt. Dabei ist der von 0 verschiedene Wert der Lichtabschwächung bei verschwindender Hornhauttiefe durch den Adhäsionsfilm der Photosensibilisatorlösung verursacht. Damit ist insbesondere die tiefenabhängige Strahlungsintensität $I(t,x,y,z)$ bekannt. Die Gültigkeit der in das Lichtabschwächungsgesetzt eingesetzten Parameter werden zudem in Abhängigkeit eines aktuellen Datenbestands aus experimentellen Versuchen an Tierhornhäuten validiert und aktualisiert. Nun kommt ein physikalisch-chemisches Berechnungsmodell zum Einsatz, welches die Polymergenerierung als Funktion der lokalen Bestrahlungsintensität $I(t,x,y,z)$ und der lokal applizierten Photosensibilisatorkonzentration $c(t,x,y,z)$ angibt. Die lokale Bestrahlungsintensität berücksichtigt hierbei die berechnete Lichtabschwächung durch den Adhäsionsfilm der (residualen) Photosensibilisatorlösung auf der Hornhaut. Somit ist die Generierung von neuen Kollagen-Polymeren durch die Wechselwirkung von UV-Strahlung und Photosensibilisator bekannt.

Darauf aufbauend kommen empirische Berechnungsmodelle zum Einsatz, welche den gewünschten Effekt auf die mechanischen Eigenschaften quantifizieren. Dies geschieht, indem die aus den physikalischen Berechnungsmodellen gewonnenen Werte der Polymererzeugung mit den Werten aus Spannungs-Dehnungsmessungen an Schweinehornhäuten unterschiedlicher Quervernetzung verknüpft werden. Fig. 5 zeigt schematisch den dabei empirisch gefundenen Zusammenhang zwischen biomechanischer Festigkeit und berechneter Polymerzahl. Eine generelle Tendenz ist dabei, dass eine höhere Polymergenerierung zu einer höheren Festigkeitszunahme führt, die durch das E-Modul der Hornhaut quantitativ beschrieben wird.

Ebenfalls aufbauend auf den Ergebnissen des physikalischen Berechnungsmodells hinsichtlich der Konzentration $c(t,x,y,z)$ und der Strahlungsintensität $I(t,x,y,z)$ wird die Keratozytenschädigungstiefe in der Hornhaut als eine der Randbedingungen bestimmt. Dazu liegen tabellierte Werte der Keratozytenschädigungstiefe vor, die in Tierversuchen gewonnen wurden.

Eine Vorhersage über die zu erwartende Krümmungsänderung als eines der Behandlungsziele wird ebenfalls simuliert und durch ein numerisches Vorhersagekriterium der Dioptrienänderung quantifiziert. Um die Nachhaltigkeit der Vorhersage weiter zu verbessern, werden das Vorhersagekriterium und weitere prä-operative Patientendaten mit bereits erzielten Krümmungsänderungen verknüpft, die ein Jahr nach Durchführung der Behandlung an einer statistisch signifikanten Vielzahl behandelter Patienten beobachtet wurden. Die Verknüpfung ist durch eine empirische Funktion gegeben, welche Ergebnis einer Korrelationsanalyse der beobachteten Krümmungsänderungen und der prä-operative Patientendaten ist. Fig. 6 zeigt schematisch die empirische Funktion, welche die nach einem Jahr erzielte Brechkraftänderung in Abhängigkeit des numerischen Vorhersagekriteriums angibt.

Werden Behandlungsziele oder Randbedingungen nicht erfüllt, so fällt im vereinfachten Verfahren eine Entscheidung gegen die Behandlung des Patienten. In der in Fig. 3 gezeigten vollumfänglichen Ausführung des Verfahrens wird die Simulation auf Grundlage einer systematischen Variation der Behandlungsparameter wiederholt. Dieser iterative Prozess wird bei Erreichen einer Obergrenze an Iterationen ebenfalls mit der Entscheidung gegen die Behandlung des Patienten abgebrochen, wenn die Behandlungszielen/Randbedingungen durch die Simulation als verfehlt vorhergesagt bleiben.

Ergibt der Vergleich der simulierten Zielgrößen mit den Randbedingungen und Behandlungszielen eine vorgegeben Übereinstimmung, so fällt die Entscheidung für die Behandlung des Patienten. Die zuletzt in der Simulation eingesetzten Behandlungsparameter stellen dabei wesentlich das gewonnene Steuerprogramm dar.

## Patentansprüche

1. Verfahren zum iterativen Erzeugen eines Steuerprogramms zum Steuern von elektromagnetischer Strahlung, mit der in Augengewebe eines Patienten eine Quervernetzung mittels eines in das Gewebe eingebrachten Photosensibilisator durchgeführt wird, um eine Änderung des Augengewebes bezüglich seiner Gestalt und/oder zumindest einer seiner mechanischen oder optischen Eigenschaften zu bewirken,
mit folgenden Schritten:

a) es werden Messdaten bezüglich des Patienten und des Auges gewonnen,
b) in einem Rechner wird mit den Messdaten und Behandlungsparametern, die ein vorgegebenes Hilfs-Steuerprogramm zum Steuern der Quelle elektromagnetischer Strahlung beinhalten, ein Ergebnis bezüglich der mit diesen Behandlungsparametern erreichten Änderung des Augengewebes simuliert,
c) die so simulierte Änderung wird mit einer zu erzielenden Änderung des Augengewebes verglichen,
d) dann, wenn Schritt c) eine hinreichende Übereinstimmung zwischen der simulierten Änderung und der zu erzielenden Änderung des Augengewebes ergibt, wird das in Schritt b) angesetzte Hilfs-Steuerprogramm als das zu erzeugende Steuerprogramm gewählt, und
e) dann wenn in Schritt c) der Vergleich keine hinreichende Übereinstimmung zwischen der simulierten Änderung und der zu erzielenden Änderung des Augengewebes ergibt, werden zumindest einer der Behandlungsparameter in Schritt b) abgeändert und damit dann die Schritte b), c) und d) erneut durchgeführt,
**dadurch gekennzeichnet, dass** in Schritt b) das Simulieren eines Ergebnisses bezüglich der erreichten Änderung des Augengewebes erfolgt durch

- Simulieren einer Diffusion des Photosensibilisators zur Bestimmung einer Verteilung des Photosensibilisators und
- Bestimmen einer tiefenabhängigen Absorption der Strahlung aufgrund der simulierten Verteilung des Photosensibilisators.

**2.** Verfahren nach Anspruch 1, **dadurch gekennzeichnet, dass** in Schritt a) als Messdaten eine oder mehrere der folgenden Daten herangezogen werden: die Hornhaut-Topographie des Auges des Patienten; die Form der Hornhautrückfläche; andere Formeigenschaften der Vorder- und/oder Rückfläche der Hornhaut, wie Vertiefungen oder Erhöhungen; biomechanische Eigenschaften der Hornhaut, wie deren Stabilität; die Hornhautdicke; die Augenlänge; die Tiefe und/oder das Volumen der Vorderkammer; Ergebnisse von Wellenfrontmessungen; das Alter; Geschlecht; hormoneller Status des Patienten; der Zustand einer gegebenen Ektasie; Lebensgewohnheiten (z.B. Rauchen) des Patienten.

**3.** Verfahren nach einem der Ansprüche 1 oder 2 **dadurch gekennzeichnet, dass** in Schritt b) das Hilfs-Steuerprogramm eine oder mehrere der folgenden Größen berücksichtigt: Intensität der Bestrahlung mit der elektromagnetischen Strahlung; Konzentration des Photosensibilisators im Gewebe; die osmotische Charakteristik des Photosensibilisators; die Bestrahlungszeit; die Zeitspanne der Applikation des Photosensibilisators vor der Bestrahlung; die Zeitspannen der Applikation des Photosensibilisators während der Bestrahlung; die Art der elektromagnetischen Strahlung (z.B. kontinuierlich oder gepulst) einschließlich der Strahlungsparameter, wie Wellenlänge; die Tastrate bei gepulster Strahlung; die räumliche und zeitliche Steuerung der Strahlung.

**4.** Computerprogrammprodukt zum Bestimmen von Parametern eines Steuerprogramms, wobei das Steuerprogramm ausgebildet ist zum Steuern einer Quelle elektromagnetischer Strahlung, mit der in Augengewebe eines Patienten eine Quervernetzung mittels eines in das Gewebe eingebrachten Photosensibilisators durchgeführt wird, um eine Änderung des Augengewebes bezüglich seiner Gestalt und/oder einer seiner mechanischen Eigenschaften zu bewirken, wobei das Computerprogrammprodukt Programmcode umfasst, welcher geeignet ist, die folgenden Schritte durchzuführen, wenn das Computerprogrammprodukt auf einer Computeranlage ausgeführt wird:

a') es werden Messdaten bezüglich des Patienten und des Auges gewonnen,
b') in einem Rechner wird mit den Messdaten und Behandlungsparametern, die ein vorgegebenes Hilfs-Steuerprogramm zum Steuern der Quelle elektromagnetischer Strahlung beinhalten, ein Ergebnis bezüglich der mit diesen Behandlungsparametern erreichten Änderung des Augengewebes simuliert,
c') die so simulierte Änderung wird mit einer zu erzielenden Änderung des Augengewebes verglichen,
d') dann, wenn Schritt c') eine hinreichende Übereinstimmung zwischen der simulierten Änderung und der zu erzielenden Änderung des Augengewebes ergibt, wird das in Schritt b) angesetzte Hilfs-Steuerprogramm als das zu erzeugende Steuerprogramm gewählt, und
b) dann wenn in Schritt c) der Vergleich keine hinreichende Übereinstimmung zwischen der simulierten Änderung und der zu erzielenden Änderung des Augengewebes ergibt, werden zumindest einer der Behandlungsparameter in Schritt b) abgeändert und damit dann die Schritte b), c) und d) erneut durchgeführt,
**dadurch gekennzeichnet, dass** in Schritt b) das Simulieren eines Ergebnisses bezüglich der erreichten Änderung des Augengewebes erfolgt durch

- Simulieren einer Diffusion des Photosensibilisators zur Bestimmung einer Verteilung des Photosensibilisators und
- Bestimmen einer tiefenabhängigen Absorption der Strahlung aufgrund der simulierten Verteilung des Photosensibilisators.

**Claims**

**1.** Process for iteratively generating a control program for controlling electromagnetic radiation, with which a cross-linking in eye tissue of a patient is implemented by means of a photosensitiser introduced into the tissue in order to bring about a change in the eye tissue with reference to its form and/or at least one of its mechanical properties, with the following steps:

a) measurement data with reference to the patient and the eye are acquired,
b) with the measurement data and treatment parameters, which involve a predetermined auxiliary control program for controlling the source of electromagnetic radiation, an outcome with reference to the change in the eye tissue obtained with these treatment parameters is simulated in a computer,
c) the change simulated in this way is compared with a change in the eye tissue to be achieved,
d) when step c) yields a sufficient agreement between the simulated change and the change in the eye tissue to be achieved, the auxiliary control program applied in step b) is chosen as the control program to be generated, and

e) when in step c) the comparison does not yield a sufficient agreement between the simulated change and the change in the eye tissue to be achieved, at least one of the treatment parameters in step b) is/are modified and steps b), c) and d) are then performed again therewith,

**characterized in that**, in step b), the simulation of an outcome with regard to the obtained change in the eye tissue is performed by

- simulating a diffusion of the photosensitizer for determining a distribution of the photosensitizer, and
- determining absorption of the radiation as a function of depth on the basis of the simulated distribution of the photosensitizer.

2. Process according to Claim 1, **characterised in that** in step a) one or more of the following data are drawn upon by way of measurement data: the corneal topography of the eye of the patient; the shape of the posterior surface of the cornea; other shape properties of the anterior and/or posterior surface of the cornea, such as depressions or elevations; biomechanical properties of the cornea, such as the stability thereof; the thickness of the cornea; the length of the eye; the depth and/or the volume of the anterior chamber; results of wavefront measurements; the age, sex, hormonal status of the patient; the state of a given ectasia; habits (e.g. smoking) of the patient.

3. Process according to one of Claims 1 or 2, **characterised in that** in step b) the auxiliary control program takes account of one or more of the following quantities: intensity of the irradiation with the electromagnetic radiation; concentration of the photosensitiser in the tissue; the osmotic characteristic of the photosensitiser; the irradiation-time; the period of application of the photosensitiser before the irradiation; the periods of application of the photosensitiser during the irradiation; the type of the electromagnetic radiation (e.g. continuous or pulsed), inclusive of the radiation parameters such as wavelength; the keying-rate in the case of pulsed radiation; the spatial and temporal control of the radiation.

4. Control program for controlling a source of electromagnetic radiation, with which a cross-linking in eye tissue of a patient is implemented by means of a photosensitiser introduced into the tissue in order to bring about a change in the eye tissue with reference to its form and/or one of its mechanical properties, the control program having being produced with the following steps:

a') measurement data with reference to the patient and the eye are acquired,
b') with the measurement data and treatment parameters, which involve a predetermined auxiliary control program for controlling the source of electromagnetic radiation, an outcome with reference to the change in the eye tissue obtained with these treatment parameters is simulated in a computer,
c') the change simulated in this way is compared with a change in the eye tissue to be achieved,
d') when step c') yields a sufficient agreement between the simulated change and the change in the eye tissue to be achieved, the auxiliary control program applied in step b') is chosen as the control program to be generated, and
e') when in step c') the comparison does not yield a sufficient agreement between the simulated change and the change in the eye tissue to be achieved, at least one of the treatment parameters in step b') is/are modified and steps b'), c') and d') are then performed again therewith,
**characterized in that**, in step b), the simulation of an outcome with regard to the obtained change in the eye tissue is performed by

- simulating a diffusion of the photosensitizer for determining a distribution of the photosensitizer, and
- determining absorption of the radiation as a function of depth on the basis of the simulated distribution of the photosensitizer.

**Revendications**

1. Procédé pour créer de manière itérative un programme destiné à commander un rayonnement électromagnétique utilisé pour réaliser une réticulation transversale dans le tissu de l'oeil d'un patient au moyen d'un photosensibilisant introduit dans ledit tissu afin d'obtenir une modification au niveau de la forme et/ou au moins d'une des propriétés mécaniques ou optiques du tissu oculaire, comportant les étapes suivantes :

a) l'acquisition de données de mesure relatives au patient et à son oeil

EP 2 334 269 B1

b) la simulation sur ordinateur, à partir desdites données de mesure et de paramètres de traitement contenant un programme de commande auxiliaire prédéterminé destiné à commander la source de rayonnement électromagnétique, d'un résultat concernant la modification du tissu oculaire obtenue à partir de ces paramètres de traitement,

c) la comparaison de la modification ainsi simulée avec une modification à réaliser du tissu oculaire

d) puis, lorsque l'étape c) permet d'établir une concordance suffisante entre la modification simulée et la modification à réaliser du tissu oculaire, la sélection du programme de commande auxiliaire utilisé à l'étape b) comme programme de commande à créer, et

e) ensuite, lorsque la comparaison effectuée à l'étape c) ne permet pas d'établir une concordance suffisante entre la modification simulée et la modification à réaliser du tissu oculaire, la modification d'au moins un des paramètres de traitement utilisé à l'étape b) et, à partir de là, la réexécution des étapes b), c) et d),

**caractérisé en ce que** la simulation, à l'étape b), d'un résultat concernant la modification obtenue du tissu oculaire est réalisée

- par simulation d'une diffusion du photosensibilisant afin de déterminer une répartition de ce dernier et
- par détermination d'une absorption du rayonnement dépendante de la profondeur, en raison de la répartition simulée du photosensibilisant.

**2.** Procédé selon la revendication 1, **caractérisé en ce qu'**une ou plusieurs des données suivantes sont utilisées comme données de mesure à l'étape a) : la topographie cornéenne de l'oeil du patient ; la forme de la surface arrière de la cornée ; d'autres propriétés de forme de la surface avant et/ou de la surface arrière de la cornée, telles que des dépressions ou des protubérances ; des propriétés biomécaniques de la cornée, telles que sa stabilité ; l'épaisseur de la cornée ; la longueur de l'oeil; la profondeur et/ou le volume de la chambre antérieure de l'oeil; des résultats de mesures de fronts d'onde ; l'âge ; le sexe ; le statut hormonal du patient ; l'état de l'ectasie rencontré ; le mode de vie du patient (p. ex. s'il fume).

**3.** Procédé selon la revendication 1 ou 2, **caractérisé en ce que** le programme de commande auxiliaire utilisé à l'étape b) prend en compte une ou plusieurs des grandeurs suivantes : l'intensité de l'irradiation par rayonnement électromagnétique ; la concentration du photosensibilisant dans le tissu ; la caractéristique osmotique du photosensibilisant ; la durée d'irradiation ; la période d'application du photosensibilisant avant l'irradiation ; les durées d'application du photosensibilisant pendant l'irradiation ; la nature du rayonnement électromagnétique (p. ex. continu ou pulsé), y compris les paramètres de rayonnement, tels que la longueur d'onde ; la fréquence d'échantillonnage du rayonnement pulsé ; la commande spatiale et temporelle du rayonnement.

**4.** Produit logiciel pour déterminer les paramètres d'un programme de commande, ce programme de commande étant conçu pour commander une source de rayonnement électromagnétique utilisée pour réaliser une réticulation transversale dans le tissu de l'oeil d'un patient au moyen d'un photosensibilisant introduit dans ledit tissu afin d'obtenir une modification au niveau de la forme et/ou au moins d'une des propriétés mécaniques ou optiques du tissu oculaire, comportant les étapes suivantes :

a') l'acquisition de données de mesure relatives au patient et à son oeil

b') la simulation sur ordinateur, à partir desdites données de mesure et de paramètres de traitement contenant un programme de commande auxiliaire prédéterminé destiné à commander la source de rayonnement électromagnétique, d'un résultat concernant la modification du tissu oculaire obtenue à partir de ces paramètres de traitement,

c') la comparaison de la modification ainsi simulée avec une modification à réaliser du tissu oculaire

d') puis, lorsque l'étape c') permet d'établir une concordance suffisante entre la modification simulée et la modification à réaliser du tissu oculaire, la sélection du programme de commande auxiliaire utilisé à l'étape b) comme programme de commande à créer, et

e') ensuite, lorsque la comparaison effectuée à l'étape c) ne permet pas d'établir une concordance suffisante entre la modification simulée et la modification à réaliser du tissu oculaire, la modification d'au moins un des paramètres de traitement utilisé à l'étape b) et, à partir de là, la réexécution des étapes b), c) et d),

**caractérisé en ce que** la simulation, à l'étape b), d'un résultat concernant la modification obtenue du tissu oculaire est réalisée

- par simulation d'une diffusion du photosensibilisant afin de déterminer une répartition de ce dernier et
- par détermination d'une absorption du rayonnement dépendante de la profondeur, en raison de la répartition simulée du photosensibilisant.

**Fig. 1**

Behandlungsparameter als
Startwerte für Optimierung

Behandlungsziele

Verfahren

Optimierte
Behandlungsparameter

Patienten-Messdaten

Patientenselektion

Randbedingungen

**Fig. 2**

Standard-
Behandlungsparameter

Patienten-Messdaten

Verfahren

Vorhersage zu Sicherheit
und Wirksamkeit

Patientenselektion

Randbedingungen

Patientenmessdaten

Behandlungsziele

Randbedingungen

Verfahren

Standard-Behandlungsparameter

Physikalische Berechnungsmodelle

Empirische Berechnungsmodelle

Zielgrössen

Vergleich der Zielgrössen mit Randbedingungen und Behandlungszielen

Systematische Variation der Behandlungsparameter

Behandlungsziel u. Randbedingung erfüllt?

JA

NEIN

(Weitere) Variation der Behandlungsparameter?

JA

NEIN

Entscheidung: Patient wird nicht behandelt

Entscheidung: Patient wird behandelt & Optimierte Behandlungsparameter

Cross-Linking Behandlung

Fig. 3

Fig. 4

Lichtabschwächung durch
Photosensibilisator und Hornhaut

Hornhauttiefe

Fig. 5

Gemessene Zunahme der
biomechanischen Festigkeit der Hornhaut

Berechnete Zunahme der
Polymerzahl in der Hornhaut

Fig. 6

Beobachtete Brechkraftänderung
der Hornhaut
ein Jahr nach Cross-Linking

Vorhersagekriterium aus gemessenen
prä-operativen Grössen

**IN DER BESCHREIBUNG AUFGEFÜHRTE DOKUMENTE**

**In der Beschreibung aufgeführte Patentdokumente**

- WO 2007128581 A2
- WO 2008000478 A1
- US 2008033408 A1
- WO 0207660 A2
- EP 2007005740 W